# EUROPEAN PATENT SPECIFICATION

(11) **EP 1 879 030 B1**
(45) Date of publication and mention of the grant of the patent: **29.04.2009**
(21) Application number: 07011811.2
(22) Date of filing: 02.03.2004
(51) Int. Cl.: G01N 33/58, G01N 33/543

(54) **Use of a virus expressing a binding moiety to measure analytes in a sample**
Verwendung eines Virus, das ein Bindungsteil exprimiert, zur Messung von Analyten in einer Probe
Utilisation d'un virus exprimant un groupe caractéristique de liaison pour mesurer des analytes dans un échantillon

(30) Priority: 04.03.2003 GB 0304832
(43) Date of publication of application: 16.01.2008
(62) Divisional of application: 04716274.8
(73) Proprietor: The Secretary of State for Defence DSTL, Salisbury, Wiltshire SP4 0JQ (GB)
(72) Inventor: Squirrrell, David James, Salisbury Wiltshire SP4 0JQ (GB); Lee, Martin Alan, Salisbury Wiltshire SP4 0JQ (GB); Mayers, Carl Nicholas, Salisbury Wiltshire SP4 0JQ (GB)
(74) Representative: Greaves, Carol Pauline

(56) References cited:
- EP-A- 0 402 997
- EP-A- 0 574 345
- US-A- 5 403 484
- US-A- 5 922 537
- US-B1- 6 265 169
- KLEIN DIETER: "Quantification using real-time PCR technology: Applications and limitations" TRENDS IN MOLECULAR MEDICINE, vol. 8, no. 6, June 2002 (2002-06), pages 257-260, XP002461653 ISSN: 1471-4914
- EDWARDS M C ET AL: "Multiplex PCR: Advantages, development, and applications" PCR METHODS AND APPLICATIONS 1994 UNITED STATES, vol. 3, no. 4, 1994, pages S65-S75, XP002461654 ISSN: 1054-9803
- EKINS R ET AL: "IMMUNOASSAY AND OTHER LIGAND ASSAYS: PRESENT STATUS AND FUTURE TRENDS" INTERNATIONAL FEDERATION OF CLINICAL CHEMISTRY. JOURNAL, vol. 9, no. 3, September 1997 (1997-09), pages 100,102-109, XP001153007

## Description

The present invention provides a novel assay method as well as kits useful in said assay.

Immuno-PCR, which dates from 1992, uses nucleic acid tagged antibodies to provide a very sensitive assay endpoint.
Generally the antibody is labelled with streptavidin and the nucleic acid through the streptavidin to the antibody. The biotinylated DNA is usually added at the end of the immunoassay.

Viruses may comprise essentially DNA or RNA, and attack host cells, integrating their nucleic acids into the host system. In structural terms, viruses generally express proteins which form a "coat" around the nucleic acids.

Phage display is a technique that was developed to allow proteins such as antibodies to be selected and produced in vitro. Phage libraries are made that contain a very high number of different proteins, such as scFv's (single chain variable fragments from antibodies). The phage has the DNA for the protein scFv's inserted into its genome and it expresses it as a fusion protein to the coat proteins, generally attached at its head.

The best protein such as scFv for a particular purpose is selected from the library mixture by "panning" for the phage that binds to the analyte of interest under strict selection conditions. The phage can then be multiplied by growth in its host bacterium and the DNA can be cut out and inserted into an expression vector. Binding protein can then be produced either as scFv or incorporated back into an antibody framework to make, for example, humanised antibodies for therapeutic use.

The phage display technique is thus used as an intermediate technique for in vitro antibody production. However, the phages themselves have never been proposed for use as assay reagents previously.

US Patent No. 6,265,169 describes a method for using filamentous bacteriophages to detect the presence of molecules of interest in biological samples.

According to the present invention there is provided a method of detecting multiple analytes in a sample, said method comprising contacting said sample with a multi-specificity mixture of viruses, each of which express and display a binding moiety, such that each virus forms a complex with an analyte or an analogue thereof, or a binding moiety for the analyte, and that the complex binds or does not bind a surface, depending upon the presence or absence of analyte in the sample, detecting the presence or absence of sequences characteristic of each virus on the surface using an amplification reaction, and relating that to the presence or absence of each analyte in the sample, and detecting on the surface the presence or absence of viral nucleic acid common to said viruses using a polymerase chain reaction.

In addition, the expression "binding moiety" relates to any moiety which will bind to a target, especially a polypeptide or protein, which may be for example an analyte polypeptide or protein, but may also be another polypeptide or protein, which is utilised in an immunoassay as part of the detection system.

The characteristic nucleic acid sequence of each virus of the multi-specificity mixture acts as a label, which may be detected using any of the known nucleic acid detection methods, in particular by using amplification reactions such as the polymerase chain reaction. However, the advantage of using a virus as compared to any other labelling technique is that a wide variety of binding moieties can be included relatively simply using techniques known for example for the production of recombinant viruses, such as phage display libraries, and without the need for additional binding steps.

Any type of viruses may be used in the context of the invention, so that thay may be DNA viruses, and in particular bacteriophages (phage), which are viruses which attack bacterial cells, but other DNA viruses or RNA viruses may also be employed. Thus, generally each virus will be a phage, and in particular a recombinant phage.

In particular, each virus will comprise a recombinant phage, which has been transformed so that expresses and displays a specific binding moiety such as an immunoglobulin for instance, an antibody, or a binding fragment thereof. However, each virus may be transformed to express any protein which may be of use in an immunoassay, including target analytes or analogues of these, even where these are not of the immunoglobulin superfamily.

Assay formats, which may use these reagents, may be any of the conventional assay forms known in immunology. For example, they may be used in both sandwich and competitive type assays.

Thus, in a particular embodiment there is provided a method as described above which comprises contacting the sample suspected of containing an analyte with a surface having immobilised thereon a binding reagent which either (a) binds each analyte, or (b) comprises each analyte or an analogue thereof, and a multi-specificity mixture of viruses which express and display binding moieties that bind either each said analyte or said binding reagent in competition to said analyte, separating said surface from the sample, and detecting the presence of said common viral nucleic acid and a nucleic acid sequence characteristic of each virus on said surface, wherein at least one of the binding reagent or the binding moiety is specific for the analyte.

Suitably, the multi-specificity mixture of viruses is incubated in the presence of the surface for a sufficient period of time to ensure that each virus binds to available binding sites on the surface, for example to any analyte present on the surface to form a bound analyte/virus complex, or to any binding reagent which is not occupied by analyte from the sample. For example, the incubation may take place for a period of from 5 to 60 minutes, at appropriate temperatures, such as from 25-40°C, for instance at about 37°C.

After this incubation, the surface including any immobilised complex is separated from the virus suspension, for example by removing the virus suspension and washing the surface.

Thereafter, a nucleic acid sequence, and in particular a nucleic acid, which may be a DNA or RNA, which is characteristic of each virus is detected on the surface.

In a sandwich type assay, the multi-specificity mixture of viruses such as the phage is selected so that each virus will bind to an analyte within a sample to form a complex. A further binding reagent for each analyte is immobilised on a surface. When the sample is contacted with the surface in the presence of the virus, complexes of analyte and virus becomes bound to the surface. This may then be separated from the residual sample. Detection of characteristic viral nucleic acid retained on the surface is indicative of the presence of the specific analyte within the sample.

In a typical competitive type assay, a binding reagent for each analyte, or the analyte or an analogue thereof, is immobilised on a surface.

As used herein, the expression "analogue" refers to a moiety that will bind to a binding partner which binds the analyte, even though it may not be of precisely the same sequence or structure as the analyte. It may, for instance, comprise a particular epitopic region of an analyte, which is bound by a specific monoclonal antibody, which therefore acts as the binding partner. Thus an analogue will "mimic" an analyte in the context of an immunoassay using a common binding partner.

A sample, to which a multi-specificity mixture of viruses including a virus that expresses and displays a binding moiety for each analyte or analogue is added, is contacted with the surface. When analyte is present in the sample, it will compete with the immobilised analyte or analogue for binding to the virus. Thus, less virus will be retained upon the surface, than would be the case if no analyte was present in the sample. This reduction in the amount of retained virus can be detected in accordance with the invention, by analysing the surface for the presence of a nucleic acid present in and characteristic of the particular virus.

In an alternative competitive type assay, a binding reagent for each analyte is immobilised on the surface. In this case, the binding moieties displayed on the viruses are each specific binding partner which are selected so that they compete with analyte for binding to the immobilised binding reagent. The less virus DNA detected on the surface after separation from the sample, the more analyte is present. Particular examples of analytes in this case are immunoglobulins such as antibodies, which may be useful in diagnosis of disease.

In all cases however, nucleic acid of each virus acts as detectable and specific "label" for the binding moiety, and may be detected using for example an amplification reaction such as a polymerase chain reaction or PCR reaction, which may be specific for the particular virus nucleic acid. Quantification of the analyte in the sample is possible using for example, quantitative PCR methods, as are well known in the art.

In particular, as discussed above, both the immobilised binding reagents and the binding moieties bind the analyte. Where analyte is present in the sample, it will form a complex in the sample. The analyte also becomes bound to the binding reagent on the surface. When the surface is removed from the sample, bound analyte/virus complex will remain, and give a positive result when characteristic viral nucleic acid is assayed for. Conversely, where the sample does not contain target analyte, virus/binding moiety, which binds to that target analyte will not become associated with the surface, and so will not be detectable.

Alternatively, each immobilised binding reagent is an analogue of an analyte, which mimics the analyte in the sense that it will bind to the binding moiety of the virus in competition with the analyte. Thus the binding moiety will bind either the analyte or the binding reagent but not both. In this case, the sample is preferably incubated with the multi-specificity mixture of viruses prior to contact with the surface. During this step, any analyte present will form a complex with the binding moiety on the virus, blocking the binding of the specific virus to the immobilised binding reagent on the surface. As a result, the complex will not be retained on the surface after washing and so the amount of detectable virus nucleic acid is reduced. In the absence of analyte, the binding moiety of the virus will be free to bind the binding reagent, resulting in a large characteristic viral nucleic acid "signal" being retained on the surface.

In some cases, each immobilised binding reagent binds an analyte, and also the binding moiety on a virus of the multi-specificity mixture. In such cases, where the analyte is present in the sample, both the analyte and the binding moiety will compete for available sites on the surface. As a result, the amount of specific virus/binding moiety that is immobilised on the surface is reduced by an amount which relates to the concentration of analyte in the sample. Again, in this case, the presence of only a lower than expected "signal" from the characteristic viral nucleic acid is indicative of the presence of analyte in the sample.

This assay can be extremely sensitive, and background signals, which are associated with conventional immunoassay methods, can be reduced. The analysis itself is more readily controlled, as each virus can be engineered to comprise whatever sequence is convenient. The detection is entirely independent upon the nature of the analyte.

The binding reagent may be any reagent that will bind to analyte.

Analytes are generally proteins or polypeptides. Typical examples will be polypeptides or proteins that are associated with or part of a pathogenic organism such as a virus, bacteria or bacterial spore such as anthrax or anthrax spores, or a protein which is indicative of a particular disease state or of exposure to a particular disease, such as an immunoglobulin for instance an antibody.

Preferably, where a binding reagent binds an analyte, it is specific for the target analyte. However, it may be relatively non-specific, for example Protein A, where the target analyte is say an immunoglobulin such as IgG, provided that a binding moiety fused to the virus is specific for the target analyte.

Suitable specific binding reagents include antibodies or binding fragments thereof, as well as lectins. Antibodies may be monoclonal or polyclonal, but are preferably monoclonal.

The binding reagents are immobilised on the surface using conventional methods. For example Protein A may be used to bind antibodies or binding fragments which include the Fc region thereof.

The surface may be any convenient surface, such as the surface of a reaction plate or well, for instance an ELISA plate or well, in addition to beads such as magnetic beads, or membranes such as cellulose membranes which are used for example in dipstick assay tests, as are conventional in the art. Where appropriate, sites which are not occupied with binding reagent may be "blocked", for example with protein such as bovine serum albumin or milk protein, or with polyvinylalcohol or ethanolamine, or mixtures of these, as is conventional in the art.

In a sandwich type assay, the sample is first incubated with the surface for a period sufficient to ensure that any analytes present become bound to the immobilised binding reagent. For example, the sample may be incubated with a blocked antibody-coated ELISA plate for a period of from 5 to 60 minutes, at appropriate temperatures, such as from 25-40°C, for instance at about 37°C.

The multi-specificity mixture of viruses may be added prior to, during or after the incubation period. Preferably however, after the incubation, residual sample is removed from the surface, which is then washed to remove any unbound analyte, before the surface is then contacted with the virus.

The multi-specificity mixture of viruses is suitably added in the form of a suspension. The surface is incubated with the virus suspension for a period of time sufficient to allow the viruses to bind to analytes on the surface. After that, excess virus suspension is removed and the surface is washed before viral nucleic acid on the surface is detected. If necessary, the viruses can be released from the surface, for example by boiling, before the detection reaction takes place.

In particular, the multi-specificity mixture of viruses used in the method is a mixture of recombinant phages which express a binding moiety for each analyte or the binding reagent that is suitably a specific binding partner. In particular, the specific binding partner will comprise a single chain variable fragment of an antibody (scFV).

Recombinant phage expressing binding moieties may be produced using conventional methods, as is well known in the production of phage libraries for phage displays as discussed above (see for example Antibody Engineering, R. Konterman & S. Dubel (eds) Springer Lab Manuals, Springer-Verlag, Berlin Heidelberg, 2001). However similar techniques may be used to produce other types of recombinant viruses.

The nucleic acid sequences of the viruses are detected using an amplification reaction, for example a polymerase chain reaction (PCR). In this case, reagents, including primers, polymerases, nucleotides, and buffers as are well known, are added to the surface, and then subjected to thermal cycling as is conventional, in order to amplify any target nucleic acid sequence present.

The amplification products may then be detected using conventional methods such as gel electrophoresis, followed by visualisation using dyes.

Preferably the amplification reactions are carried out in such a way that the amplification product generates a detectable signal, and in particular a visible signal, for example a fluorescent signal, as it progresses. Many assay formats that produce such signals are known in the art. They may utilise reagents such as DNA binding agents such as intercalating dyes which emit radiation and particularly fluorescent radiation at greater intensity when they are intercalated into double stranded DNA, as well as probes and primers which include fluorescent labels, arranged to undergo fluorescent energy transfer (FET) and particularly fluorescent resonant energy transfer (FRET).

There are two commonly used types of FET or FRET probes, those using hydrolysis of nucleic acid probes to separate donor from acceptor, and those using hybridisation to alter the spatial relationship of donor and acceptor molecules.

Hydrolysis probes are commercially available as TaqMan^{™} probes. These consist of DNA oligonucleotides that are labelled with donor and acceptor molecules. The probes are designed to bind to a specific region on one strand of a PCR product. Following annealing of the PCR primer to this strand, *Taq* enzyme extends the DNA with 5' to 3' polymerase activity. *Taq* enzyme also exhibits 5' to 3' exonuclease activity. TaqMan^{™} probes are protected at the 3' end by phosphorylation to prevent them from priming *Taq* extension. If the TaqMan^{™} probe is hybridised to the product strand, an extending *Taq* molecule may also hydrolyse the probe, liberating the donor from acceptor as the basis of detection. The signal in this instance is cumulative, the concentration of free donor and acceptor molecules increasing with each cycle of the amplification reaction.

Hybridisation probes are available in a number of forms. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacons to a complementary sequence the fluorescent labels are separated, so FRET does not occur, and this forms the basis of detection.

Pairs of labelled oligonucleotides may also be used. These hybridise in close proximity on a PCR product strand-bringing donor and acceptor molecules together so that FRET can occur. Enhanced FRET is the basis of detection. Variants of this type include using a labelled amplification primer with a single adjacent probe.

Other methods for detecting amplification reactions as they occur are known however, and any of these may be used. Particular examples of such methods are described for example in WO 99/28500, British Patent No. 2,338,301, WO 99/28501 and WO 99/42611.

WO 99/28500 describes a very successful assay for detecting the presence of a target nucleic acid sequence in a sample. In this method, a DNA duplex binding agent and a probe specific for said target sequence, is added to the sample. The probe comprises a reactive molecule able to absorb fluorescence from or donate fluorescent energy to said DNA duplex binding agent. This mixture is then subjected to an amplification reaction in which target nucleic acid is amplified, and conditions are induced either during or after the amplification process in which the probe hybridises to the target sequence. Fluorescence from said sample is monitored.

An alternative form of this assay, which utilises a DNA duplex binding agent which can absorb fluorescent energy from the fluorescent label on the probe but which does not emit visible light, is described in co-pending British Patent Application No. 223563.8. Any of these assays may be used in the context of the assay method of the invention in order to detect the target nucleic acid sequence.

Many of these assays can be carried out in a quantitative manner as is well known in the art, for example by monitoring the signal from the amplification mixture at least once during each cycle of the amplification reaction. By carrying out the reaction in this way, the amount of virus present on the surface may be determined, and this may be related to the amount of analyte present in the original sample.

The particular characteristic sequence of the virus detected may be any characteristic sequence found therein such as the sequence encoding the scFv itself, or a specifically introduced marker sequence.

If desired, specific marker sequences may be introduced into the virus at the same time as the binding moiety, for example fused to the sequence encoding the binding moiety, or may be added in a separate transformation operation.

Sequences common to viruses or recombinant viruses, such as phage DNA or RNA, or antibiotic resistance genes, arealso detected. Generally, there will always be some carry-over of viral nucleic acid, which can be used as internal reference sequences, ensuring that the PCR reaction has proceeded appropriately.

In this case multiplex PCR reactions using different signalling reagents or systems may be employed in order to detect the various sequences which are produced. This may be achieved, for example by labelling probes or primers used in the amplification reaction using different labels, for example, labels which fluoresce at different wavelengths. Examination of the signal from each label, for example at each of the different wavelength, is then carried out, if necessary with appropriate signal resolution where the wavelengths overlap.

Alternatively the assay is designed such that the amplicons produced by different PCR reactions hybridise to form duplexes or destabilise at different temperatures. Melting point analysis, for example using intercalating dyes that exhibit increased fluorescence when bound to double stranded DNA species, is a well-known technique. By adding such as dye to the reaction system, either during or after the assay process, and by monitoring fluorescence with a controlled change of temperature, the temperature at which the duplex structure of the amplicon breaks down or reforms can be determined, and this can be related to the presence of the particular amplicon and hence the particular virus which has bound.

The assay system of the invention thus provides a useful and reliable assay method.

Kits for use in the assay method described above form a further aspect of the invention.

In particular, the invention provides a kit for detecting multiple analytes in a sample comprising the surface and the multi-specificity mixture of viruses as described above, and probes for carrying out the detecting steps described above.

For instance, where the surface has immobilised thereon binding reagents which binds analytes, each virus of the multi-specificity mixture suitably expresses and displays a binding partner for an analyte, or a binding partner which binds said binding reagent in competition to ananalyte.

Alternatively, where the surface has immobilised thereon binding reagents which comprises either the analyte or an analogue thereof, each virus of the multi-specificity mixture is suitably one which expresses and displays a binding partner for an analyte.

Suitably, the surface is of a solid body that is a well in a plate, for instance a multi-well plate, but it may also be beads such as magnetic beads, or membranes, for example cellulose membranes as found in conventional dipstick type assays.

Possible additional elements of the kit comprise reagents suitable for use in the detection of the nucleic acid sequences. In particular, the kit may comprise intercalating dyes or primers for use the detection of the particular nucleic acid sequences as discussed above. For example, the kit may comprise primers which amplify sequences, which encode specific scFv sequences, or marker sequences which have been incorporated into the virus. In addition, the kits may include primers which are suitable for amplifying virus sequences, sequence which encode scaffolding of scFvs or antibiotic resistance genes which are present in recombinant virus.

The primers may suitably be labelled in such a way that the amplification product is directly detectable. For example, they may include fluorescent or other labels as described above.

The kits include probes, which are specific for the amplification products and which are labelled to assist in detection of products. They may comprise single- or dual-labelled hydrolysis or hybridisation probes, also as discussed above. When appropriate they may include intercalating dyes or other DNA duplex binding agents, which form elements of the detection system.

Kits may also include intercalating dyes to assist with melting point analysis, where this is required in order to resolve multi-specificity assay results.

The methods described above will now be illustrated with reference to the accompanying drawings in which:
Figure 1 illustrates diagrammatically, a sandwich assay that may occur in the course of the method of the invention;
Figure 2 illustrates diagrammatically a competitive assay that may occur in the course of the method of the invention;
Figure 3 is a graph of fluorescence -d(F1)/dt versus temperature when carrying out a PCR reaction of the TAQMAN® type;
Figure 4 is a graph of fluorescence (F1) against cycle number of series of samples at different dilutions using the assay of the invention; and
Figure 5 shows the results of an assay described hereinafter for *B. cereus* spores including a PCR for detecting phage DNA.

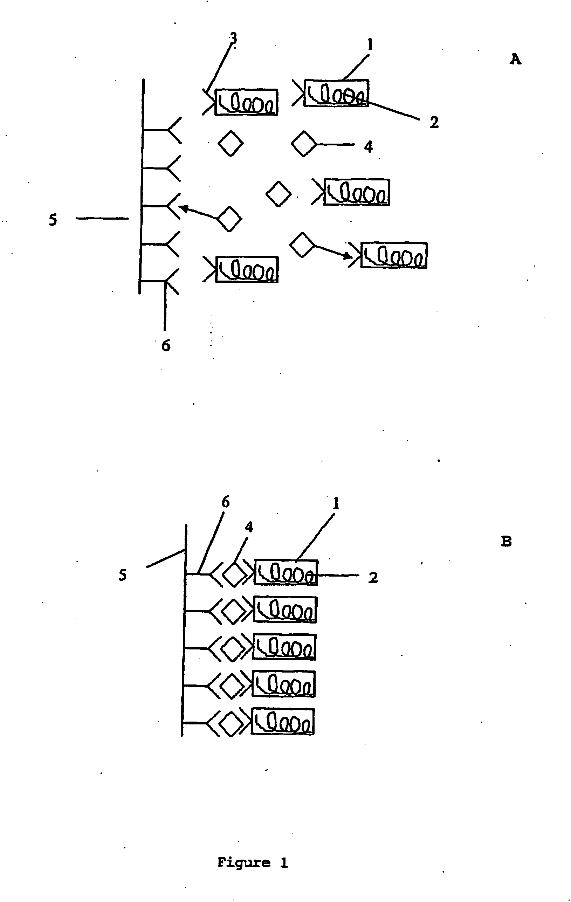

In the sandwich assay illustrated in Figure 1A, a phage (1) is used, which comprises an outer coat, enclosing phage DNA (2). A binding partner (3) such as an scFv is expressed by the phage and displayed on the surface at the head of the phage. It is added as a reagent to an assay reaction mixture which may contain analyte (4), and is in contact with a surface, such as a bead or well (5) on which an antibody (6) which is also specific for the analyte (4) is immobilised.

On incubation (B), the phage (1) and analyte (4) forms a complex which is retained on the surface (5), by the binding of the analyte to the antibody (6). Thereafter, the surface (5) is removed from the remainder of the sample and washed. However, some phage is retained on the surface, where it may be detected.

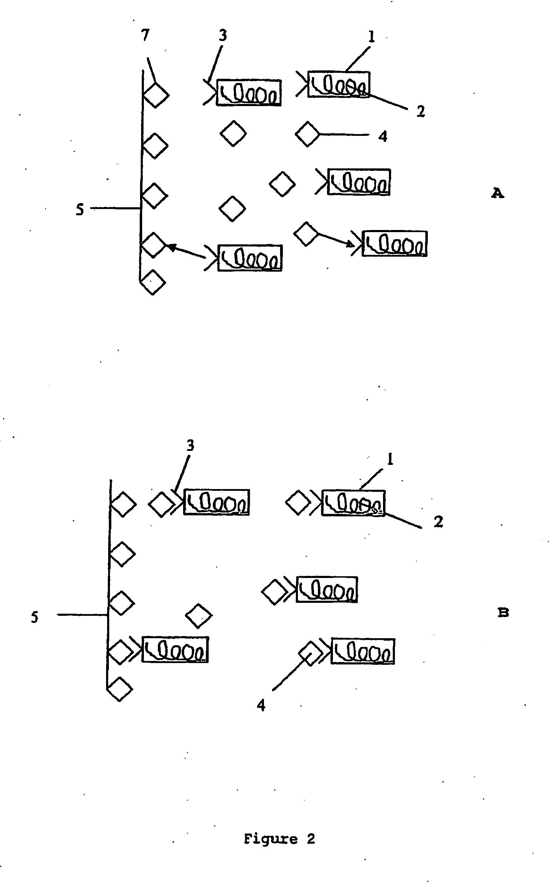

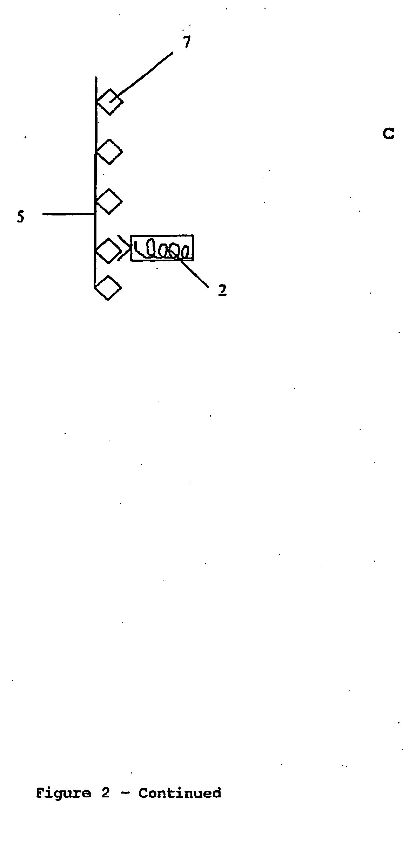

In the embodiment illustrated in Figure 2A, an analyte or an analogue thereof (7) capable of binding to the binding partner (3) of the phage (1) is immobilised on the surface (5). A sample under test to which the phage (1) has been added is incubated in the presence of this surface. Analyte (4) in the sample will bind to the binding partner (3) of the phage (1). Any phage which has undergone such binding is unable to bind to the immobilised analyte analogue (7) (B), and therefore will be washed away with the sample during a subsequent separation step. Detection of phage DNA (2) on the surface (5) following such a washing step will reveal lower levels than would otherwise be expected if no analyte were present.

### Illustration 1

### Demonstration of Assay using Bacillus cereus spores

### 1) Plate format

A sample (50µl) comprising a suspension of *B.cereus* spores (1 x 10⁸ per ml) in distilled sterile water was added to each well of a blocked ELISA plate (Immulon microtitre ELISA plate) which was then placed in an oven at 37°C overnight to dry the spores onto the plates.

The plates were then washed three times with a wash solution comprising 0.05% v/v Tween 20 in phosphate buffered saline (PBS) or PBST.

A blocking buffer (200ml) comprising 2% w/v dry milk powder and 0.05% v/v Tween 20 PBS was added to each well. The plate was then sealed and incubated at room temperature for a minimum of 1 hour. It was then washed again three times in PBST.

A solution of primary antibody expressing M13 phage (1x 10⁹ transforming units per ml), wherein the primary antibody is a *B.cereus* specific single chain variable fragment (scFv), in PBST blocking buffer was prepared and at least 50µl added per well. PBST blocking buffer was added to one of the wells in place of the primary antibody expressing phage as a negative control.

The plate was incubated at 37°C for 1 hour, then washed 5 times in PBST. After drying, 50 µl dH₂O was added to each well. The plate was then boiled for 30 seconds to free the phage for PCR.

After allowing the plate to cool briefly, and contents of the wells were transferred to a PCR tube, together with a conventional PCR mix (18 µl) including M13 phage specific primers and SybrGreen, used in accordance with the manufacturer's instructions.

The sample subjected to a series of thermal cycling steps on the Roche LightCycler as follows:
94°C for 0 seconds (melt);
62°C for 30 seconds (annealing phase);
72°C for 30 seconds (extension phase).

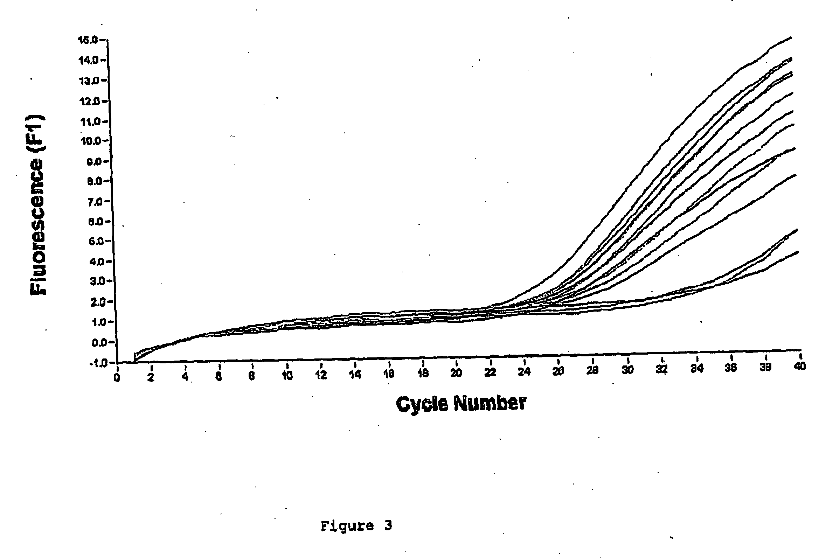

40 cycles were carried out. The fluorescent signal from the samples was monitored once per cycle at the end of the extension phase. The process was repeated with an increasingly dilute sample and the results are shown in Figure 3.

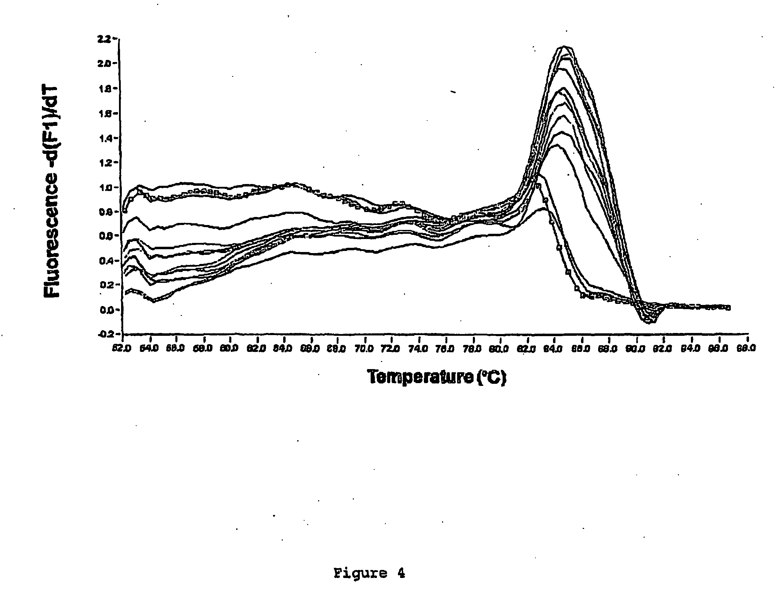

Negative control samples showed only a small increase in signal at the end of the cycling process. It was confirmed by a final melt curve analysis (Figure 4) that the signal from the negative control was due to non-specific products such as primer-dimers.

The results show however that the presence of bacterial spores in the samples was detectable using this method.

### Illustration 2

### Detection Assay

For use as a detection assay, a sample is added to a blocked-antibody coated ELISA plate and incubated at 37°C for 5 to 60 minutes.

Thereafter, the plate is washed with wash liquid from three to five times.

A suspension of filamentous phage expressing an scFv specific for the assay target is added to the plate, and incubated for 5-60 mins. After further washing (3-5 times), conventional PCR reagents are added, together with a suitable reporter system such as the SybrGreen dye mentioned above. However, other reporter mechanisms, for example using fluorescent reporter probes, such as TAQMAN^{®} or other probes for in situ monitoring may be employed. The reaction mixture is thermally cycled to effect the amplification in the conventional way.

The PCR cycle number at which product appears (fluorescence threshold crossing point) is noted and correlated with the concentration of analyte in original sample.

It is possible to add more than one scFv with different specificities at the same time, to determine a range of targets. In such cases, melt profiles may be carried out to distinguish which one of the scFv is present and therefore has bound to the analyte. If desired, phage sequences or antibiotic resistance sequences found in the transformed phage may be used as an internal reference for the PCR.

### Illustration 3

### Alternative Filtration Assay Format

In this embodiment, a liquid sample is passed through a 0.2 or 0.45 micron filter, depending upon the nature of the assay target, and the filter is then washed. Target analyte, for example bacterial spores, are retained on the filter. Subsequently, a suspension of filamentous phage expressing an scFv specific for the assay target is also passed through the filter, which is again washed. Any phage which binds to the target on the filter can then be detected, by PCR as described above.

### Illustration 4

### Detection of phage displaying single chain antibodies directed against B cereus in an immunoassay format.

50µl of 10⁷ *B. cereus* spores/ml were diluted 10 fold in dH₂O down an Immulon 2 ELISA plate spores and dried onto the plate at 37°C overnight. This immobilised the spores on the plate so that they mirrored the situation in which an analyte was binding an immobilised antibody, as might occur in an assay for the spores.

The plate was then washed in dH₂O, three times. Each well was then blocked by the addition of 150µl of 1% blotto/phosphate buffered saline (PBS) and the plate was then incubated at 37 °C for 1 hour. The plate was washed in PBS-Tween, three times. 50µl of phage suspension in 1% blotto/PBS was added to each well and then the plate was incubated at 37°C for one hour, so that the phage bound to the spores on the plate. The plate was then washed in PBS-Tween 3 times, followed by three washes in dH₂O to remove unbound phage.

50µl of dH₂O was then added to each well and the plate was heated in boiling water for 30 seconds so as to elute the phage.

2µl from each well were then assayed by PCR using phage directed primers, amplify the lacI gene found within the M13 derived phage. Real-time PCR was performed using a Corbett RotorGene. Each tris-buffered reaction contained 0.5µM each of forward and reverse LacI primers, 0.3 µM of lacI specific TaqMan probe, 4 mM MgCl₂. Cycling parameters were 95° for 5 seconds and 60°C for 1 minute for 50 cycles. The primers, probe and target were as follows:

| | |
|---|---|
| *FORWARD PRIMER* | *5'-CGTGGTGGTGTCGATGGTAG* |
| *REVERSE PRIMER* | *5'-TGTGCACCGCTTT* |
| *PROBE SEQUENCE* | *5'- ACGAAG CGGCGTCGAA GCCTG* |
| AMPLICON | 5'-CGTGGTGGTGTCGATGGTAGAACGA AGCGGC GTCGAAGCCTGTAAGCGGCGGTGCACA |

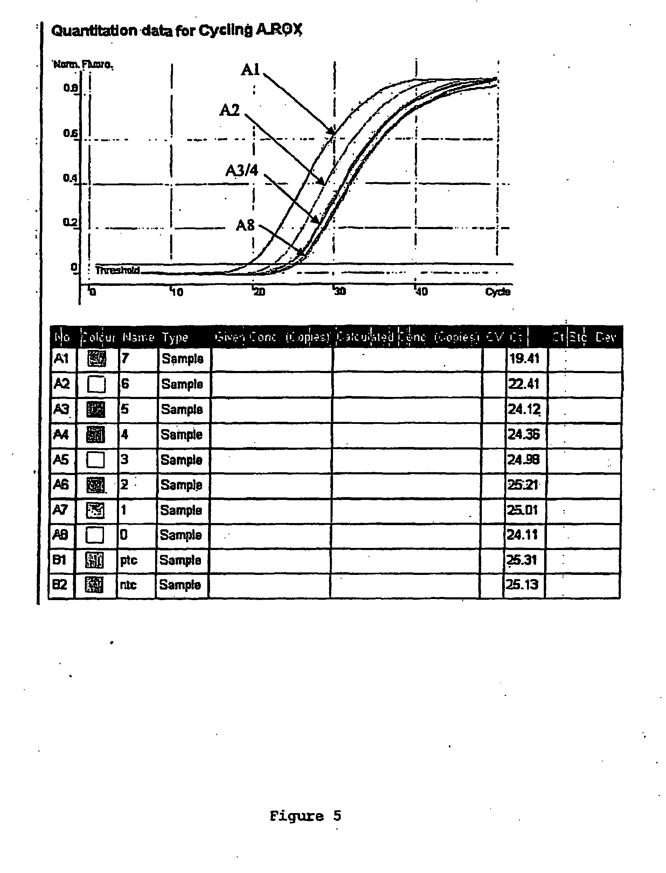

The results are shown in Figure 5. The results show that 10⁶ to 10³ spores per well were detectable above background level, even though the background level in this case was quite high. This was probably as a result of cross-contamination. There are shared genes between M13 derived phages and M13 derived cloning vectors used routinely in the lab. The sensitivity of the assay could be readily improved by setting up the phage PCR reaction in a lab that is free of M13 contamination or by choosing primers specific to phages displaying *B. cereus* antibodies.

## Claims

1. A method of detecting multiple analytes in a sample, said method comprising contacting said sample with a multi-specificity mixture of viruses, each of which express and display a binding moiety, such that each virus forms a complex with an analyte or an analogue thereof, or a binding moiety for the analyte, and that the complex binds or does not bind a surface, depending upon the presence or absence of analyte in the sample, detecting the presence or absence of sequences characteristic of each virus on the surface using an amplification reaction, and relating that to the presence or absence of each analyte in the sample, and detecting on the surface the presence or absence of viral nucleic acid common to said viruses using a polymerase chain reaction.

2. A method according to claim 1 which comprises contacting the sample suspected of containing an analyte with a surface having immobilised thereon a binding reagent which either (a) binds each analyte, or (b) comprises each analyte or an analogue thereof, and a multi-specificity mixture of viruses which express and display binding moieties that bind either each said analyte or said binding reagent in competition to said analyte, separating said surface from the sample, and detecting the presence of said common viral nucleic acid and a nucleic acid sequence characteristic of each virus on said surface, wherein at least one of the binding reagent or the binding moiety is specific for the analyte.

3. A method according to claim 2 where both the immobilised binding reagent and the binding moieties bind an analyte, so that a complex comprising the binding reagent, the analyte and the virus is retained on the surface after separation of the sample therefrom, and the presence of characteristic viral nucleic acid on the surface is indicative of the presence of analyte in the sample.

4. A method according to claim 2 where the immobilised binding reagent comprises the analytes or an analogue thereof, so that the binding moieties will bind either the analytes or the binding reagent, and the presence of analytes in the sample blocks the binding of the binding moiety to the binding reagent, so that a reduction in the amount of each virus able to bind to the binding reagent is indicative of the presence of corresponding analyte in the sample.

5. A method according to claim 2 wherein the binding reagent binds the analyte, and also the binding moiety on each virus, so that the analyte and the binding moiety will compete for available sites on the surface, so that a reduction in the amount of each virus able to bind to the binding reagent is indicative of the presence of corresponding analyte in the sample.

6. A method according to any one of claims 1 to 5 wherein the binding reagent is a specific binding reagent.

7. A method according to any one of claims 1 to 6 wherein the surface is the surface of an ELISA plate or well, a magnetic bead or a membrane.

8. A method according to any one of claims 1 to 7 wherein sites on the surface which are not occupied with binding reagent are blocked.

9. A method according to claim 8 in which the following steps are carried out sequentially:
i) sample is incubated with the surface for a period sufficient to ensure that any analytes present becomes bound to the immobilised binding reagent,
ii) residual sample is removed from the surface, which is then washed to remove any unbound analyte,
iii) the surface is contacted with a suspension of the multi-specificity mixture of viruses, and incubated for a period of time sufficient to allow the virus to bind to analyte on the surface;
iv) virus suspension is removed and the surface is washed; and
v) nucleic acid characteristic of each virus and sequences common to viruses or recombinant viruses on the surface is detected.

10. A method according to claim 9 wherein the virus nucleic acid is released from the surface before step (v).

11. A method according to any one of claims 1 to 10 wherein each virus is a recombinant phage which expresses a specific binding moiety which specifically binds either the analyte or a specific binding partner for the analyte.

12. A method according to claim 11 wherein the specific binding partner is a single chain variable fragment of an antibody (scFV).

13. A method according to any one of the preceding claims wherein the amplification reaction is a polymerase chain reaction (PCR).

14. A method according to any one of the preceding claims wherein the amplification reaction is carried out in such a way that the amplification product generates a detectable signal.

15. A method according to claim 14 wherein the signal is a visible signal.

16. A method according to claim 14 or claim 15 wherein the amplification reaction is carried out in the presence of a DNA binding reagent, or a primer or probe which is labelled with a fluorescent label.

17. A method according to claim 16 wherein the DNA binding agent is an intercalating dye.

18. A method according to any one of the preceding claims wherein the amount of a virus detected is quantified, and this is related to the amount of corresponding analyte in the sample.

19. A method according to any one of the preceding claims wherein a nucleic acid sequence characteristic of a particular virus used in the method is a marker sequence which has been introduced into the virus.

20. A method according to any one of the preceding claims wherein viral nucleic acid common to viruses or recombinant viruses detected are phage DNA, RNA or antibiotic resistance gene sequences.

21. A method according to any one of the preceding claims wherein a subsequent melting point analysis is conducted to determine which virus has bound during the assay.

22. A method according to any one of the preceding claims wherein the viruses are RNA viruses.

23. A kit for detecting multiple analytes in a sample comprising the surface and the multi-specificity mixture of viruses specified in claim 1, and probes for carrying out the detecting steps specified in claim 1.

24. A kit according to claim 23 wherein each virus is a recombinant phage which expresses a specific binding moiety for either said analyte or said binding reagent in competition to said analyte.

25. A kit according to any one of claims 23 or 24 which further comprises an intercalating dye.

## Patentansprüche

1. Verfahren zum Nachweisen von mehrfachen Analyten in einer Probe, wobei das Verfahren folgendes umfasst: das Kontaktieren der Probe mit einem Multispezifitätsgemisch von Viren, von denen jedes einen Bindungsrest exprimiert und anzeigt, so dass jedes Virus einen Komplex mit einem Analyten oder einem Analogen davon bildet, oder einen Bindungsrest für den Analyten bildet und dass der Komplex an eine Oberfläche bindet oder nicht bindet, und zwar in Abhängigkeit von der Anwesenheit oder Abwesenheit eines Analyten in der Probe, das Nachweisen der Anwesenheit oder Abwesenheit von Sequenzen, die für jedes Virus charakteristisch sind, an der Oberfläche unter Anwendung einer Amplifikationsreaktion, und das Inbeziehungsetzen dieses Ergebnisses mit der Anwesenheit oder Abwesenheit eines jeden Analyten in der Probe und das Nachweisen der Anwesenheit oder Abwesenheit einer viralen Nucleinsäure, die für diese Viren üblich ist, unter Anwendung einer Polymerase-Kettenreaktion auf der Oberfläche.

2. Verfahren nach Anspruch 1, umfassend das Kontaktieren der Probe, bei der ein Analytgehalt vermutet wird, mit einer Oberfläche, auf der ein Bindungsreagens immobilisiert ist, das (a) entweder die einzelnen Analyten bindet oder (b) die einzelnen Analyten oder ein Analoges davon umfasst, und eines Multispezifitätsgemisches von Viren, die Bindungsreste, die entweder die einzelnen Analyten binden oder das Bindungsreagens in Konkurrenz mit dem Analyten umfassen, exprimieren und anzeigen, das Abtrennen der Oberfläche von der Probe und das Nachweisen der Anwesenheit der üblichen viralen Nucleinsäure und einer Nucleinsäuresequenz, die für die einzelnen Viren an der Oberfläche charakteristisch ist, wobei mindestens einer der Bestandteile Bindungsreagens oder Bindungsrest für den Analyten spezifisch ist.

3. Verfahren nach Anspruch 2, wobei sowohl das immobilisierte Bindungsreagens als auch die Bindungsreste einen Analyten binden, so dass ein Komplex, der das Bindungsreagens, den Analyten und das Virus umfasst, an der Oberfläche nach Trennung der Probe davon zurückgehalten wird, und wobei die Gegenwart einer charakteristischen viralen Nucleinsäure auf der Oberfläche ein Anzeichen für die Anwesenheit des Analyten in der Probe darstellt.

4. Verfahren nach Anspruch 2, wobei das immobilisierte Bindungsreagens die Analyten oder ein Analoges davon umfasst, so dass die Bindungsreste entweder die Analyten oder das Bindungsreagens binden, und wobei die Anwesenheit von Analyten in der Probe die Bindung des Bindungsrestes an das Bindungsreagens blockiert, so dass eine Verringerung der Menge der einzelnen Viren, die zur Bindung an das Bindungsreagens befähigt sind, ein Anzeichen für die Anwesenheit des entsprechenden Analyten in der Probe darstellt.

5. Verfahren nach Anspruch 2, wobei das Bindungsreagens den Analyten und auch den Bindungsrest an den einzelnen Viren bindet, so dass der Analyt und der Bindungsrest um die verfügbaren Stellen an der Oberfläche konkurrieren, so dass eine Verringerung der Menge der einzelnen Viren, die zur Bindung an das Bindungsreagens befähigt sind, ein Anzeichen für die Anwesenheit des entsprechenden Analyten in der Probe darstellt.

6. Verfahren nach einem der Ansprüche 1 bis 5, wobei es sich beim Bindungsreagens um ein spezifisches Bindungsreagens handelt.

7. Verfahren nach einem der Ansprüche 1 bis 6, wobei es sich bei der Oberfläche um die Oberfläche einer ELISA-Platte oder -Vertiefung, ein Magnetkügelchen oder eine Membran handelt.

8. Verfahren nach einem der Ansprüche 1 bis 7, wobei die Stellen an der Oberfläche, die nicht vom Bindungsreagens besetzt sind, blockiert werden.

9. Verfahren nach Anspruch 8, wobei die folgenden Stufen nacheinander durchgeführt werden:
i) Probe wird mit der Oberfläche für eine ausreichende Zeitspanne inkubiert, um zu gewährleisten, dass etwaige vorhandene Analyten an das immobilisierte Bindungsreagens gebunden werden,
ii) restliche Probe wird von der Oberfläche entfernt, die anschließend zur Entfernung von etwaigem ungebundenem Analyten gewaschen wird,
iii) die Oberfläche wird mit einer Suspension des Multispezifitätsgemisches von Viren in Kontakt gebracht und für eine ausreichende Zeitspanne inkubiert, um dem Virus die Bindung an den Analyten an der Oberfläche zu ermöglichen;
iv) Virussuspension wird entfernt und die Oberfläche wird gewaschen; und
v) Nucleinsäure, die für die einzelnen Viren charakteristisch ist, und für Viren und rekombinante Viren auf der Oberfläche übliche Sequenzen werden nachgewiesen.

10. Verfahren nach Anspruch 9, wobei die Virus-Nucleinsäure vor der Stufe (v) von der Oberfläche freigesetzt wird.

11. Verfahren nach einem der Ansprüche 1 bis 10, wobei es sich bei den einzelnen Viren um rekombinante Phagen handelt, die einen spezifischen Bindungsrest exprimieren, der spezifisch entweder an den Analyten oder einen spezifischen Bindungspartner für den Analyten bindet.

12. Verfahren nach Anspruch 11, wobei es sich beim spezifischen Bindungspartner um ein einzelkettiges variables Fragment eines Antikörpers (scFV) handelt.

13. Verfahren nach einem der vorstehenden Ansprüche, wobei es sich bei der Amplifkationsreaktion um eine Polymerase-Kettenreaktion (PCR) handelt.

14. Verfahren nach einem der vorstehenden Ansprüche, wobei die Amplifikationsreaktion so durchgeführt wird, dass das Amplifikationsprodukt ein nachweisbares Signal erzeugt.

15. Verfahren nach Anspruch 14, wobei es sich bei dem Signal um ein sichtbares Signal handelt.

16. Verfahren nach Anspruch 14 oder 15, wobei die Amplifikationsreaktion in Gegenwart eines DNA-Bindungsreagens oder eines Primers oder einer Sonde, die mit einer fluoreszierenden Markierung markiert sind, durchgeführt wird.

17. Verfahren nach Anspruch 16, wobei es sich beim DNA-Bindungsreagens um einen interkalierenden Farbstoff handelt.

18. Verfahren nach einem der vorstehenden Ansprüche, wobei die Menge eines nachgewiesenen Virus quantitativ bestimmt wird und diese Menge mit der Menge des entsprechenden Analyten in der Probe in Beziehung gesetzt wird.

19. Verfahren nach einem der vorstehenden Ansprüche, wobei eine Nucleinsäuresequenz, die für ein bestimmtes, im Verfahren verwendetes Virus charakteristisch ist, eine Markersequenz ist, die in das Virus eingeführt worden ist,

20. Verfahren nach einem der vorstehenden Ansprüche, wobei es sich bei der viralen Nucleinsäure, die für nachgewiesene Viren oder rekombinante Viren üblich ist, um Phagen-DNA, RNA oder antibiotische Resistenzgensequenzen handelt.

21. Verfahren nach einem der vorstehenden Ansprüche, wobei eine anschließende Schmelzpunktsanalyse durchgeführt wird, um festzustellen, ob ein Virus während des Tests gebunden worden ist.

22. Verfahren nach einem der vorstehenden Ansprüche, wobei es sich bei den Viren um RNA-Viren handelt.

23. Kit zum Nachweis von mehrfachen Analyten in einer Probe, umfassend die Oberfläche und das Multispezifitätsgemisch von Viren gemäß Anspruch 1 sowie Sonden zur Durchführung der in Anspruch 1 angegebenen Nachweisstufen.

24. Kit nach Anspruch 23, wobei es sich bei den einzelnen Viren um rekombinante Phagen handelt, die einen spezifischen Bindungsrest entweder für den Analyten oder das Bindungsreagens in Konkurrenz mit dem Analyten exprimieren.

25. Kit nach einem der Ansprüche 23 oder 24, das ferner einen interkalierenden Farbstoff umfasst.

## Revendications

1. Procédé de détection d'analytes multiples dans un échantillon, ledit procédé comprenant la mise en contact dudit échantillon avec un mélange de virus à spécificités multiples, dont chacun exprime et présente un radical de liaison, de telle manière que chaque virus forme un complexe avec un analyte ou un analogue de celui-ci, ou un radical de liaison pour l'analyte, et que le complexe se lie ou ne se lie pas à une surface, selon la présence ou l'absence de l'analyte dans l'échantillon, la détection de la présence ou l'absence de séquences caractéristiques de chaque virus sur la surface en utilisant une réaction d'amplification, et la relation de cela à la présence ou l'absence de chaque analyte dans l'échantillon, et la détection sur la surface de la présence ou de l'absence d'acide nucléique viral commun auxdits virus en utilisant une réaction en chaîne de la polymérase.

2. Procédé selon la revendication 1, qui comprend la mise en contact de l'échantillon suspecté de contenir un analyte avec une surface ayant immobilisé dessus un réactif de liaison qui soit (a) lie chaque analyte soit (b) comprend chaque analyte ou un analogue de celui-ci, et un mélange de virus à spécificités multiples qui expriment et présentent des radicaux de liaison qui lient soit chaque dit analyte soit ledit réactif de liaison en compétition avec ledit analyte, la séparation de ladite surface de l'échantillon, et la détection de la présence dudit acide nucléique viral commun et d'une séquence d'acide nucléique caractéristique de chaque virus sur ladite surface, où au moins l'un du réactif de liaison ou du radical de liaison est spécifique de l'analyte.

3. Procédé selon la revendication 2 où à la fois le réactif de liaison immobilisé et les radicaux de liaison lient un analyte, de telle manière qu'un complexe comprenant le réactif de liaison, l'analyte et le virus est retenu sur la surface après la séparation de l'échantillon de celle-ci, et la présence d'acide nucléique viral caractéristique sur la surface est indicative de la présence de l'analyte dans l'échantillon.

4. Procédé selon la revendication 2 où le réactif de liaison immobilisé comprend les analytes ou un analogue de ceux-ci, de telle manière que les radicaux de liaison lieront soit les analytes soit le réactif de liaison, et la présence des analytes dans l'échantillon bloque la liaison du radical de liaison au réactif de liaison, de telle manière qu'une réduction de la quantité de chaque virus capable de se lier au réactif de liaison est indicative de la présence de l'analyte correspondant dans l'échantillon.

5. Procédé selon la revendication 2, dans lequel le réactif de liaison lie l'analyte, et également le radical de liaison sur chaque virus, de telle manière que l'analyte et le radical de liaison entreront en compétition pour les sites disponibles sur la surface, de telle manière qu'une réduction de la quantité de chaque virus capable de se lier au réactif de liaison est indicative de la présence de l'analyte correspondant dans l'échantillon.

6. Procédé selon l'une quelconque des revendications 1 à 5, dans lequel le réactif de liaison est un réactif de liaison spécifique.

7. Procédé selon l'une quelconque des revendications 1 à 6, dans lequel la surface est la surface d'une plaque ou d'un puits ELISA, d'une bille magnétique ou d'une membrane.

8. Procédé selon l'une quelconque des revendications 1 à 7, dans lequel les sites sur la surface qui ne sont pas occupés avec le réactif de liaison sont bloqués.

9. Procédé selon la revendication 8, dans lequel les étapes suivantes sont réalisées séquentiellement :
i) l'échantillon est incubé avec la surface pendant une période suffisante pour assurer que tous les analytes présents se retrouvent liés au réactif de liaison immobilisé,
ii) l'échantillon résiduel est retiré de la surface, qui est ensuite lavée pour éliminer tout analyte non lié,
iii) la surface est mise en contact avec une suspension du mélange de virus à spécificités multiples, et incubée pendant une période de temps suffisante pour permettre au virus de se lier à l'analyte sur la surface ;
iv) la suspension de virus est retirée et la surface est lavée ; et
v) l'acide nucléique caractéristique de chaque virus et des séquences communes aux virus ou aux virus recombinants sur la surface est détecté.

10. Procédé selon la revendication 9, dans lequel l'acide nucléique du virus est libéré de la surface avant l'étape (v).

11. Procédé selon l'une quelconque des revendications 1 à 10, dans lequel chaque virus est un phage recombinant qui exprime un radical de liaison spécifique qui lie spécifiquement soit l'analyte soit un partenaire de liaison spécifique de l'analyte.

12. Procédé selon la revendication 11, dans lequel le partenaire de liaison spécifique est un fragment variable à chaîne unique d'un anticorps (scFV).

13. Procédé selon l'une quelconque des revendications précédentes, dans lequel la réaction d'amplification est une réaction en chaîne de la polymérase (PCR).

14. Procédé selon l'une quelconque des revendications précédentes, dans lequel la réaction d'amplification est réalisée d'une telle manière que le produit de l'amplification génère un signal détectable.

15. Procédé selon la revendication 14, dans lequel le signal est un signal visible.

16. Procédé selon la revendication 14 ou la revendication 15, dans lequel la réaction d'amplification est réalisée en présence d'un réactif de liaison d'ADN, ou d'une amorce ou d'une sonde qui est marqué avec un marqueur fluorescent.

17. Procédé selon la revendication 16, dans lequel l'agent de liaison d'ADN est un colorant intercalant.

18. Procédé selon l'une quelconque des revendications précédentes, dans lequel la quantité d'un virus détecté est quantifiée, et celle-ci est reliée à la quantité d'analyte correspondant dans l'échantillon.

19. Procédé selon l'une quelconque des revendications précédentes, dans lequel une séquence d'acide nucléique caractéristique d'un virus particulier utilisé dans le procédé est une séquence marqueur qui a été introduite dans le virus.

20. Procédé selon l'une quelconque des revendications précédentes, dans lequel l'acide nucléique viral commun aux virus ou aux virus recombinants détectés est de l'ADN de phage, de l'ARN ou des séquences de gènes de résistance à un antibiotique.

21. Procédé selon l'une quelconque des revendications précédentes, dans lequel une analyse subséquente du point de fusion est conduite pour déterminer quel virus s'est lié au cours du test.

22. Procédé selon l'une quelconque des revendications précédentes, dans lequel les virus sont des virus à ARN.

23. Kit de détection d'analytes multiples dans un échantillon comprenant la surface et le mélange de virus à spécificités multiples spécifiés dans la revendication 1, et des sondes pour réaliser les étapes de détection spécifiées dans la revendication 1.

24. Kit selon la revendication 23, dans lequel chaque virus est un phage recombinant qui exprime un radical de liaison spécifique soit dudit analyte soit dudit réactif de liaison en compétition avec ledit analyte.

25. Kit selon l'une quelconque des revendications 23 ou 24, qui comprend en outre un colorant intercalant.
